# EUROPÄISCHE PATENTANMELDUNG

(11) **EP 1 375 494 A1**
(43) Veröffentlichungstag der Anmeldung: **02.01.2004**
(21) Anmeldenummer: 03013884.6
(22) Anmeldetag: 20.06.2003
(51) Int. Cl.: C07D 323/00, C07D 405/06, C07D 407/06, C07H 17/08, C07F 7/12, C07F 7/18, C07F 7/08, C09K 3/00

(54) **Tetraetherlipide mit kleinen Kopfgruppen, deren Herstellung und Verwendung**

(30) Priorität: 26.06.2002 DE 10228857
(71) Anmelder: Surface & Interface Technologies GmbH, 37308 Heilbad Heiligenstadt (DE)
(72) Erfinder: Bakowsky, Udo, Dr., 99817 Eisenach (DE); Kneuer, Carsten, Dr., 04451 Borsdorf (DE); Rothe, Ulrich, Dr., 06193 Frössnitz (DE)
(74) Vertreter: Pauling, Hans-Jürgen

(57) **Zusammenfassung**

1. Tetraetherlipide mit kleinen Kopfgruppen, deren Herstellung und Verwendung.
2.1. Tetraetherlipide sind bipolare Amiphile, ursprünglich aus natürlichen Bakterien isoliert. Sie werden verwendet für sphärische Vesikel, pharmazeutische Formulierungen u.a.. Jedoch sind aus thermophilen Mikroorganismen stammende Tetraetherlipide in ihrer natürlichen Kopfgruppenstruktur nicht zur kovalenten Anbindung geeignet und bisherige Isolationsmethoden weisen nicht zufriedenstellende Ausbeuten auf.
   Aufgabe der Erfindung ist es, stabile und zugleich reaktionsfähige Tetraetherlipide mit einem verbesserten Verfahren zu erzeugen.
2.2. Die vorliegende Erfindung betrifft Tetraetherlipide mit kleinen Kopfgruppen und aktivierte Tetraetherlipide mit kleinen Kopfgruppen sowie ein Verfahren zur Herstellung der genannten Tetraetherlipide.
   Danach werden bei archaebakteriellen Lipid-Rohextrakten vollständig die natürlichen Kopfgruppen abgespalten und nachfolgend gereinigt.
2.3. Die erfindungsgemäße Lösung ist insgesamt eine qualitative Weiterentwicklung zur Herstellung und Anwendung von Tetraetherlipiden für Oberflächenbeschichtungen und Kompositmaterialien.

## Beschreibung

Die Erfindung betrifft Tetraetherlipide mit kleinen Kopfgruppen sowie aktivierte Tetraetherlipide, deren Herstellung und Verwendung.

Tetraetherlipide (nachfolgend als TEL abgekürzt) sind bipolare membrandurchspannende Amiphile, die ursprünglich aus natürlichen Quellen (Archaebakterien) isoliert wurden. TEL besitzen eine bemerkenswerte chemische und biologische Stabilität und sind in der Lage, sich an Grenzflächen zu monomolekularen Schichten zu organisieren oder Vesikel zu bilden.
Unbehandelte archaebakterielle TEL bestehen chemisch aus einem 72-gliedrigen Makroetherzyklus, in dem die Kohlenstoffatome der Phytanylketten kovalent miteinander verbunden sind. Die beiden hydrophilen Kopfgruppen bestehen aus verschiedenen asymmetrisch angeordneten chemischen Gruppierungen. Typischerweise ist die eine Gruppe ein Phosphorsäurederivat, die andere stellt eine Zuckerkomponente dar. Thermoplasma acidophilum wird bei einem pH-Wert von 2 und Temperaturen zwischen 39 und 59°C in Fermentern gezüchtet. Die Lipidgewinnung erfolgt nach Extraktion und anschließende chromatographische Trennung. Die Struktur des Kettensegments ist, bedingt durch den Zellstoffwechsel, nicht einheitlich. In Abhängigkeit von der Züchtungstemperatur werden in unterschiedlichen Maße Pentazyklisierungen in das Kettensegment eingebaut.

Aus dem Stand der Technik ist weiter bekannt, derartige TEL als Reinstoff sowie in Mischung mit herkömmlichen Phospholipiden zur Herstellung sphärischer Vesikel zu verwenden und für den Einsatz zur Herstellung pharmazeutischer Formulierungen vorzusehen. Weiterhin wurden in die erhaltenen Vesikel Proteine inkorporiert und deren Funktionsweise getestet (vgl. WO 97/22333, WO 97/31297 und WO 99/10337). Bekannt sind auch weitere Verwendungen, wie Beschichtung planarer Membranen, chromatographische Trennungen u.a. analytische Zwecke.

Jedoch sind aus thermophilen Mikroorganismen stammende TEL in ihrer natürlichen Kopfgruppenstruktur nicht zur kovalenten Anbindung geeignet und die bisher bekannten Isolationsmethoden weisen nicht zufriedenstellende Ausbeuten auf.

Der Erfindung liegt deshalb die Aufgabe zu Grunde, stabile und zugleich reaktionsfähige Tetraetherlipide zu erzeugen. Eine weitere Aufgabe der Erfindung besteht in der Entwicklung eines verbesserten Verfahrens zur Herstellung dieser Tetraetherlipide.

Diese Aufgabe wird erfindungsgemäß gelöst durch die Bereitstellung von TEL mit der allgemeinen Formel A gemäß Patentanspruch 1.
Die Reste R und R₁ sind darin, unabhängig voneinander, Gruppen gem. 1.1. bis 1.15. sowie durch Bildung von Pentazyklen im Tetraethergrundgerüst gebildete Modifikationen davon.

In einer weiteren bevorzugten Ausführungsform gemäß Patentanspruch 1 wird ein TEL der allgemeinen Formel A bereitgestellt, darin wird der Rest R aus den Gruppen 2.1. bis 2.3. gebildet und der Rest R₁ aus den Gruppen 1.1. bis 1.15. gemäß Patentanspruch 1 ausgewählt.

Die erfindungsgemäßen TEL mit der allgemeinen Formel A werden durch ein Verfahren gemäß Patentanspruch 3 hergestellt.
Danach werden bei den biotechnologisch gewonnenen archaebakteriellen Lipid-Rohextrakten vollständig die natürlichen Kopfgruppen durch Umsetzen mit Pyridinhydrochlorid oder durch Hydrolyse mittels Halogenwasserstoffen abgespalten und das erhaltene Produkt nachfolgend gereinigt.

Die Aufgabe der Erfindung wird weiter durch aktivierte TEL mit kleinen Kopfgruppen der allgemeinen Formel B gemäß Patentanspruch 4 gelöst.
Darin sind die Reste R₃ und R₄, unabhängig voneinander,
4.1. ―CH₂-OH oder ―CH₂-ONa
4.2. ―CH₂-OH oder -CH₂-Tos
4.3. ―CH₂-OH oder -CH₂-I

Die erfindungsgemäßen TEL gemäß den Patentansprüchen 1 und 2 sowie die aktivierten TEL gemäß Patentanspruch 4 werden zu Oberflächenbeschichtungen und/oder zur Herstellung von Komposit-Materialien verwendet.

Die erfindungsgemäße Lösung ist insgesamt eine qualitative Weiterentwicklung der Erforschung, Herstellung und Anwendung von Tetraetherlipiden.

Die Erfindung wird nachstehend anhand von Beispielen näher erläutert.

### Beispiel 1:

### Herstellung von TEL aus der Rohlipidfraktion von Thermoplasma acidophilum.

1.1. Die Kultivierung von Thermoplasma acidophilum erfolgt in Fermentern, wobei die etablierten Wachstumsbedingungen Verwendung finden. Die Ernte wird wie üblich durchgeführt.
   Die Extraktion der Gesamtlipide erfolgte aus der getrockneten, homogenisierten Zellmasse unter kontinuierlichem Rückfluss. Dazu wurde die Rohmasse mit ca. 30 Gewichtsequivalenten Lösungsmittel (Chloroform/Methanol 2:1, v:v) versetzt und 12 Stunden unter Rückfluss erhitzt. Die Ausbeute der Extraktion betrug 90% des Gesamtlipidanteils.
   Die Darstellung der erfindungsgemäßen TEL erfolgte aus der Rohlipidfraktion durch Abspaltung aller polaren Kopfgruppen. Dieses Verfahren ermöglichte eine Erhöhung der Ausbeute an TEL sowie eine wesentliche Vereinfachung des technologischen Ablaufes durch die Einsparung aufwendiger Trennschritte.
   Zur Abspaltung von Kopfgruppen der Rohlipidfraktion stehen 3 Verfahren zur Verfügung. Bei allen Methoden kommt es sowohl zu einer Aufspaltung der vorhandenen Esterbindungen wie auch der in 3-Stellung am Glycerol vorhandenen Etherbindung. Die Etherbindungen in 1- bzw. 2-Stellung am Glycerol werden dabei wietestgehend von einer Spaltung verschont.
1.2.1. Umsetzung mit Pyridinhydrochlorid
   Die über Phosphorpentoxid getrocknete Rohlipidfraktion wurde mit der equimolaren Menge Pyridinhydrochlorid verrieben. Das Gemisch wurde in einem sekurrierten, luftdichten Rundkolben unter Verwendung eines Metallbades geschmolzen. Bei einer konstanten Temperatur von 110°C erfolgte die Abspaltung der Kopfgruppen innerhalb von 6 Stunden.
   Nach Abschluss der Reaktion wurde die Schmelze zermahlen und mit 500 ml Wasser/g Rohextrakt versetzt und intensiv geschüttelt. Anschließend wurden
   600 ml Chloroform/Methanol (9:1, v:v) je g Rohextrakt hinzugegeben und das Lipid gelöst. Die organische Phase wurde abgetrennt. Der Vorgang wurde zweimal wiederholt. Die erhaltenen organischen Fraktionen wurden vereinigt und am Rotationsverdampfer eingeengt. Die so erhaltene Rohausbeute an TEL betrug ca. 50% des Rohextraktgewichtes.
1.2.2. Hydrolyse mit Bromwasserstoff
   Die über Phosphorpentoxid getrocknete Rohlipidfraktion wurde in 600 ml Toluol/Benzol (60:40, v:v) je g Rohextrakt gelöst und mit 200 ml 48% wässriger HBr-Lösung/g Rohextrakt versetzt. Das so entstandene zweiphasige Reaktionsgemisch wurde am Rückfluss unter ständigem Rühren für 12 Stunden bis zum Sieden erhitzt.
   Nach Abschluss der Reaktion wurde die organische Phase abgetrennt. Anschließend wurde die organische Phase dreimal mit jeweils 600 ml/g Rohextrakt Wasser gewaschen. Dabei wurde solange Natriumcarbonat zugesetzt, bis das Waschwasser neutral reagiert. Anschließend wurde die lipidhaltige Lösung an einem Rotationsverdampfer eingeengt. Die so erhaltene Rohausbeute an TEL betrug ca. 66% des Rohextraktgewichtes.
1.2.3. Hydrolyse mit lodwasserstoff
   Die über Phosphorpentoxid getrocknete Rohlipidfraktion wurde in 600 ml Toluol/Benzol (60:40, v:v) je g Rohextrakt gelöst. Anschließend wurde gasförmiger lodwasserstoff bis zur Sättigung eingeleitet. Das Gemisch wurde am Rückfluss unter Rühren für sechs Stunden auf 80°C erhitzt.
   Nach Abschluss der Reaktion wurde die organische Phase fünfmal mit jeweils 600 ml Wasser/g Rohextrakt gewaschen. Dabei wurde solange Natriumcarbonat zugesetzt, bis das Waschwasser neutral reagiert. Anschließend wurde die lipidhaltige Lösung am Rotationsverdampfer eingeengt. Die so erhaltene Rohausbeute an TEL betrug ca. 60% des Rohextraktgewichtes.
1.3. Reinigung der nach 1.2. erhaltenen Reaktionsprodukte
   Die chromatographische Fraktionierung des Reaktionsansatzes erfolgte mit einem Radial-Chromatographen. Dazu wurde die Reaktionsmischung in 30 Gewichtsäquivalenten Chloroform gelöst und auf die zirkuläre Kieselgelscheibe (3 mm dick) des verwendeten Chromatographen aufgetragen. Anschließend wurde mit jeweils 25 Gewichtsäquivalenten Lösungsmittelgemisch im Gradientenverfahren beginnend von einem Verhältnis Chloroform/Methanol 1:5 (v:v) bis 10:1 (v:v) kontinuierlich eluiert. Die erhaltenen Fraktionen wurden gesammelt und analysiert. Fraktionen mit TEL wurden vereinigt und am Rotationsverdampfer eingeengt. Die Ausbeute betrug ca. 62% bezogen auf die Masse an eingesetztem Reaktionsgemisch.
1.4. Überprüfung der Identität und Reinheit der gereinigten Produkte
   Die Identifizierung der TEL erfolgte mittels Dünnschichtchromatographie (TLC). Die nach 1.3. gereinigten Reaktionsprodukte wurden in Chloroform/Methanol (2:1, v:v) gelöst und eine geeignete Menge auf eine Kieselgelplatte aufgetragen. Als Laufmittel diente Chloroform/Methanol (9:1, v:v). Zur Visualisierung der TEL-Bande wurde die Veraschungsmethode mit methanolischer Schwefelsäure sowie die lodfärbung verwendet. Für das TEL nach der allgemeinen Formel A, wobei R und R₁ = CH₂OH bedeuten, wurde ein Rf-Wert von 0,7 gefunden. Sowohl die Elementaranalyse, die Massenspektrometrie als auch die NMR erbrachten identische Ergebnisse zur Referenz.

### Beispiel 2:

### Aktivierung des TEL-Grundgerüstes

2.1. Bildung von Tosylprodukten
   150 mg (10⁻⁴ mol) des Tetraethers wurden in 20 ml trockenem Pyridin gelöst. 2x10⁻⁴ mol Tosylchlorid wurden in 7 ml Pyridin gelöst und unter Eiskühlung, Rühren und Feuchtigkeitsausschluss dieser Lösung zugegeben. Danach wurde 20 min unter Kühlung und weitere 24 Stunden bei Raumtemperatur weitergerührt. Die Ausbeute an beidseitig aktiviertem Produkt lag bei 63% (mittels DC). Um die Umsetzung zu vervollständigen, wurde erneut die o.g. Menge an Tosylchlorid zugegeben und die beschriebene Prozedur wiederholt. Die produkthaltige Lösung wird schließlich eingeengt und mittels Dünnschichtchromatographie (Chloroform:Methanol = 9:1, v:v) gereinigt.
   Der Nachweis der Umsetzung erfolgte mittels FTIR Spektroskopie, die für den gebundenen Tosyl-Rest charakteristische Banden bei 980, 1100, 1180, 1610 cm⁻¹ zeigt. Die Rf-Werte in der Dünnschichtchromatographie für das einseitig aktivierte und das beidseitig aktivierte Produkt waren 0,78 bis 0,9.
2.2. Bildung von lodprodukten
   150 mg (10⁻⁴ mol) eines Toluensulfonsäuretetraethers und 40 mg (2,67 x 10⁻² mol) Natriumiodid wurden in 10 ml trockenem Aceton unter Licht- und Feuchtigkeitsausschluss dispergiert. Anschließend wurde für 24 Stunden unter Rückfluss bis zum Sieden erhitzt. Nach Abschluss der Reaktion wurde das Aceton abdestilliert und der verbleibende Rückstand mit Chloroform aufgenommen. Der unlösliche Anteil wurde abfiltriert. Danach wurde die chloroformische Lösung des Produktes dreimal mit jeweils 20 ml 3%iger Thiosulfatlösung gewaschen. Anschließend wurde die produkthaltige Lösung eingeengt und mittels Plattenchromatographie (Chloroform/Methanol = 9:1, Silofor 0,5 mm dick) DC-rein dargestellt. Die Ausbeute des doppelseitig aktivierten Produktes betrug mindestens 48% (HPTLC). Der Nachweis der Umsetzung erfolgte mittels FTIR-Spektroskopie, wobei das Verschwinden der Tosyl typischen Banden bei 980, 1100, 1180 und 1610 cm⁻¹ zu beobachten war. Massenspektrum und Elementaranalyse waren mit den berechneten Werten konform. Der Rf-Wert für das beidseitig aktivierte Produkt betrug 0,86 (Siliciumdioxid, Chloroform/Methanol = 9:1, v:v), die entsprechende Bande war lod und Brommethylblau positiv.
   Das lodprodukt kann zur Synthese weiterer Verbindungen der allgemeinen Formel ―CH-NRₓR_{y} verwendet werden.

### Beispiel 3:

### Herstellung von TEL mit kleinen Kopfgruppen

3.1. Herstellung eines Thiolproduktes
   150 mg eines Toluensulfonsäuretetraethers wurden in 50 ml Chloroform gelöst und mit 80 mg KSH (gelöst in 25 ml Ethanol) unter ständigem Rühren vermischt. Anschließend wurde unter Rückfluss für 24 Stunden bis zum Sieden erhitzt. Nach dem Abkühlen wurde weitere 24 Stunden nachgerührt. Das Lösungsmittel wurde abdestilliert und der verbleibende Rückstand mehrmals mit Wasser gewaschen. Das verbleibende Rohprodukt wurde in 50 ml Chloroform gelöst und dreimal mit jeweils 25 ml Wasser gewaschen. Anschließend wurde die produkthaltige organische Phase eingeengt und mittels Plattenchromatographie (Chloroform/Methanol = 9:1, Kieselgel 0,5 mm dick) DC-rein dargestellt. Die Ausbeute betrug mindestens 32% des doppelseitig aktivierten Produktes (HPTLC). Die l-dentifizierung erfolgte mittels FTIR-Spektroskopie, wobei das Verschwinden der Tosyl-typischen Banden sowie das Auftreten der SH-Bande verfolgt wurde. Die Elementaranalyse war mit den berechneten Werten konform. Der Rf-Wert betrug 0,72 (Siliciumdioxid, Chloroform/Methanol = 9:1, v:v)- die entsprechende Bande war lod und Brommethylblau positiv und die Thiolgruppe konnte mit dem N(2-iodacetoxy)ethyl-N-methylamino-7-nitrobenzoxa-1,3-diazol) Test nachgewiesen werden.

## Patentansprüche

1. Tetraetherlipide mit kleinen Kopfgruppen der allgemeinen Formel A, darin sind R und R₁, unabhängig voneinander, 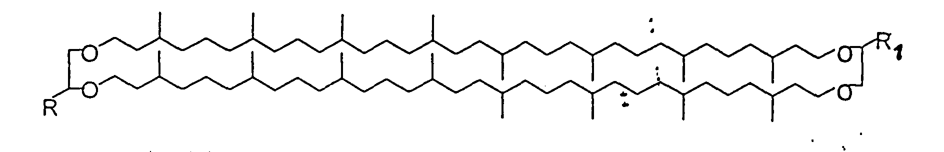
1.1. ―CH2-I oder -CH₂-IO₃
1.2. ―CH₂-Br
1.3 ―CH₂-ONa
1.4 ―[CH₂-NX₃]⁺, wobei X ein Alkan oder eine aromatische Verbindung ist
1.5 ―CH₂-Pyridiniumderivate
1.6 ―CH₂-Tosylate
1.7 ―CH₂-SCN
1.8 ―CH₂-N=C=O
1.9 ―CH₂-N=N-H
1.10 -CH₂-Ethylenoxirane
1.11 -CH₂-N₃
1.12 Silane, der allgemeinen Formel: (A)-Si(B)b(C)c,
wobei A aus der Gruppe
-CH₂-
-CH₂-O-
- CH₂-O-(CH₂)x-, (x=1-30)
- CH₂-O-(CH₂)x-O-, (x=1-30)
- CH₂-(CH₂)x-O-, x=1-30 stammt,
B aus folgender Gruppe ausgewählt wird
-Cl
-Br
-I
-F
-OH
und C folgende Bedeutung haben kann
-O-CH₃
-O-CH₂-CH₃
-CH₃
-O-Phenyl
-O-Benzyl
-O-CF₃
-O-CF₂-CF₃
-CF₃
- Glucose,-Mannose,-Galactose und andere Glycoside
1.13 halogenierte Alkanyle, Alkenyle und Alkanylether, sowie Alkenylether insbesondere jedoch CH2-CF3, CH2-CF2-CF3, CH2-O-CF2-CF3
1.14 derivatisierte Alkanyle und Alkanylether der allgemeinen Formel - CH₂-(A)a-(B) oder -CH₂-O-(A)a-(B) oder CH₂-NH-(A)a-(B), wobei a=0-30
und A ausgewählt wird aus der Gruppe
CH₂
CH₂-O-
CH₂-CH₂-O
CF₂
CF₂-O
CF₂-CF₂-O
Und B sein kann:
-OH
-NH₂
-SH
-COOH
-CO-Cl
- CO-NH₂
-CH₃
-CF₃
- CH=CH₂
-CCH
-CF=CF₂
-CCF
-SCN
-CN
-N=NH
-N₃
-N(X)₃, wobei X = -CH₃, -O-CH₃ oder ―O-CH₂-CH₃
weiterhin kann B bedeuten: Pyridin, Phenol, Benzen, Mono-, Di- und Polysaccharide, Biotin, Peptide, Proteine, Antikörper, Avidine, Lektine oder Oligonucleotide
sowie durch Bildung von Pentazyklen im Tetraethergrundgerüst gebildete Modifikationen davon.

2. Tetraetherlipide mit kleinen Kopfgruppen der allgemeinen Formel A, darin sind R 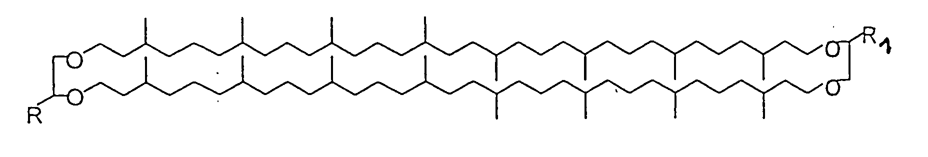
-CH₂-NH₂
-CH₂-OH
- CH₂-Cyanurchlorid
- CH₂-SH
-COCl
und der Rest R₁ wird aus den Gruppen 1.1. bis 1.14. gemäß Patentanspruch 1 ausgewählt.

3. Verfahren zur Herstellung von Tetraetherlipiden mit kleinen Kopfgruppen gemäß den Patentansprüchen 1 und 2 aus einem biotechnologisch gewonnenen archae bakteriellen Lipidrohextrakt, **gekennzeichnet durch** eine vollständige Abspaltung aller natürlichen Kopfgruppen **durch** Umsetzung mit Pyridinhydrochlorid oder Hydrolyse mittels Halogenwasserstoffen und nachfolgender Reinigung.

4. Aktivierte Tetraetherlipide mit kleinen Kopfgruppen der allgemeinen Formel B, darin sind R₃ und R₄ unabhängig voneinander 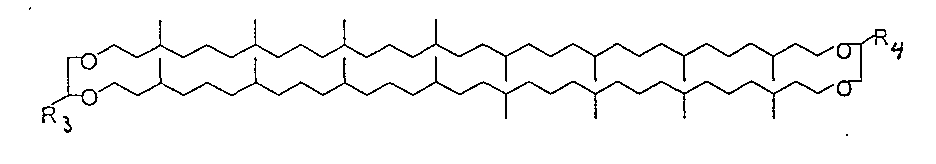
4.1. ―CH₂-OH oder ―CH₂-ONa
4.2. ―CH₂-OH oder ―CH₂-Tos
4.3. ―CH₂-OH oder ―CH₂-l

5. Verwendung von Tetraetherlipiden mit kleinen Kopfgruppen gemäß den Patentansprüchen 1 und 2 zur Oberflächenbeschichtung und/oder Herstellung von Kompositmaterialien.

6. Verwendung von aktivierten Tetraetherlipiden gemäß Patentanspruch 4 zur Oberflächenbeschichtung und/oder Herstellung von Kompositmaterialien.
